# EUROPEAN PATENT APPLICATION

(11) **EP 0 816 507 A1**
(43) Date of publication of application: **07.01.1998**
(21) Application number: 96906030.0
(22) Date of filing: 15.03.1996
(51) Int. Cl.: C12N 15/86, A61K 48/00

(54) **NOVEL GENE RECOMBINANT**

(30) Priority: 16.03.1995 JP 84921/95
(71) Applicant: HISAMITSU PHARMACEUTICAL CO. INC., Tosu-shi Saga 841 (JP)
(72) Inventor: SHIMADA, Takashi, Tokyo 113 (JP); AKIYAMA, Katsuhiko, Tsukuba Laboratory,, Kannondai, Tsukuba-shi, Ibaraki 305 (JP); SUZUKI, Yousuke, Tsukuba Laboratory,, Kannondai, Tsukuba-shi, Ibaraki 305 (JP)
(74) Representative: VOSSIUS & PARTNER
(86) International application number: JP9600658
(87) International publication number: WO9628565

(57) **Abstract**

A recombinant human immunodeficiency virus vector which carries a DNA sequence encoding a cytotoxin.

## Description

### TECHNICAL FIELD

This invention relates to a recombinant human immunodeficiency virus vector and a process for producing the same. More particularly, it relates to a novel recombinant human immunodeficiency virus vector which is useful in gene therapy for diseases caused by CD4-positive cells and a process for producing the same.

### BACKGROUND ART

Acquired immunodeficiency syndrome (AIDS) is a disease caused by infection with human immunodeficiency virus (HIV) in which cellular immunity is significantly damaged and thus various opportunistic infections, lymphomas, nervous disorders, etc. are induced. Remedies *for HIV employed at the present stage are drugs called nucleotide reverse transcriptase inhibitors exemplified by azidothymidine (AZT), didanosine (ddI), zalcitabine (ddC), etc. which are used either alone or in combination. However, these drugs cannot eliminate cells which have been already infected. Moreover, there arise serious problems including potent bone marrow depression, severe side effects such as digestive disorders, the appearance of drug-tolerant viruses, etc. Therefore, it has been urgently required to develop a drug of a novel type which has enhanced efficacy with few side effects or tolerance therefor.

The term "gene therapy" refers to a completely novel treatment for diseases through the expression of a foreign gene which has been introduced into the body and is capable of acting as a drug. It is expected that gene therapy will be efficacious against all of congenital and acquired diseases caused by abnormalities in genes. In particular, it is expected that gene therapy will be highly useful in treating cancer and AIDS which are fatal diseases and for which no therapy has been established so far. In the United States, attempts have been already initiated to apply gene therapy to the treatment of heredity diseases [adenosine deaminase (ADA) deficiency, low density lipoprotein (LDL) receptor deficiency, cystic lung fibrosis, etc.] and cancer (brain tumor, malignant melanoma, etc.). In recent years, a number of fundamental studies have been made on gene therapy for viral infectious diseases typified by AIDS.

Prospective candidates for gene therapy for AIDS with the use of drug genes include a method wherein Tat or Rev, which seemingly promote the replication of HIV, is inhibited by using RNA decoys such as TAR decoy or RRE decoy [Sullenger B.A., et al., Cell, 63, 601 (1990)]; a method wherein mRNA or DNA of HIV is hybridized with an antisense oligonucleotide [Chatterjee S., et al., Science, 258, 1485 (1992)]; a method wherein RNA of HIV is cleaved with a ribozyme [Stevelo K.M., et al., Virology, 190, 176 (1992)]; and a method wherein the function of a protein essentially required in the replication of HIV is inhibited by a transdominant mutant [Hope T. J., et al., J. Virol., 66, 1849 (1992)]. Although the replication mechanism of HIV is skillfully utilized in these therapeutic systems, it is required therein that the expression of the drug genes predominates over the replicability of HIV. Accordingly, it seems that further work is needed to develop promoters aimed at more efficient gene expression.

Recently, studies have been also made on the introduction of an enzyme gene capable of converting a non-toxic prodrug into a toxic one. When modified in cells via phosphorylation into the triphosphate, ganciclovir [i.e., a guanosine analog; 9-(1,3-dihydroxy-2-propoxymethyl)guanine, hereinafter referred to simply as GCV] is incorporated into DNA similar to usual nuclear bases. Then elongation of the DNA in the cells is terminated immediately thereafter, thus causing cellular death. Two types of enzymes are needed in the phosphorylation for forming the triphosphate. First, the monophosphorylation of GCV is catalyzed by thymidine kinase originating in a virus which is an enzyme observed exclusively in virus-infected cells. That is to say, this phosphorylation never occurs in cells which are not infected with a virus. To the GCV monophosphate, two additional phosphate groups are further added by a phosphorylase originating in cells and thus GCV triphosphate is formed. GCV is known as an antiviral agent depending on these function mechanisms. Recently, these systems are employed in the field of gene therapy. Namely, it has been attempted to treat cancer by introducing genes encoding thymidine kinase originating in virus into cancer cells followed by the induction of cellular death with the use of GCV, i.e., as means for eliminating the cancer cells from the body [Shu-Hsia Chen, et al., Proc. Natl. Acad. Sci. USA, 91, 3054 (1994)].

Also, attempts are made to apply this therapeutic system to gene therapy for AIDS. Venkatesh et al. constructed a recombinant adenovirus vector by ligating thymidine kinase gene to the downstream of HIV LTR and inserting these genes into the genome structure of an adenovirus [Venkatesh L.K., et al., Proc. Natl. Acad. Sic. USA, 87, 8746 (1990)].

On the other hand, the efficiency of the transfer of a drug gene into the target cells is an important factor in improving the therapeutic achievement, similar to the development of the drug genes as described above. There have been made various attempts to transfer genes at elevated efficiency. Although it was attempted to employ physical procedures such as microinjection early in the 1980's, only a poor transfer efficiency could be established and genes could not be transferred in a stable state thereby. Furthermore, the limited techniques for cell incubation on a mass scale in those days made it impossible to put such attempts into practical use. Subsequently, there were developed recombinant viruses (virus vectors) for efficiently transferring foreign genes into target cells, which made it possible for the first time to apply gene therapy for clinical purposes.

There are several types of virus vectors as will be described hereinbelow. The virus vectors most frequently employed in gene therapy today are retrovirus vectors originating in moloney murine leukemia virus (MoMLV). That is to say, genes are transferred by taking advantage of the proliferation manner of this virus. A retrovirus is an RNA virus having an envelope which invades cells through the bond of the envelope protein to the receptor in the host cell side. After the invasion, the single-stranded virus RNA is converted into a double-stranded DNA via a reverse transcriptase and is thus integrated into the genomic DNA of the infected cells in a stable state, though at random. However, the integration cannot be completed unless the cells are dividing and proliferating [Miller D.G., et al., Molecular and Cellular Biology, 10 (8), 4239 (1990)]. The retrovirus gene thus integrated is called a provirus. From this provirus, RNA is transcribed and thus viral proteins are synthesized. Then new viral particles are formed from these proteins and the virus RNA. In a retrovirus vector, the retrovirus gene in the above-mentioned case recombines with a foreign gene [Miller A.D., Current Topics in Microbiology and Immunology, 158, 1 (1992)]. As it is accepted over a wide range of hosts, this MoMLV vector enables gene transfer into various cells. With respect to its safety, a number of studies have been carried out hitherto and no serious problem has arised so far [Kenneth C., Br. H. Hematol., 80, 421 (1992)].

Also, adenovirus vectors may be cited as another example of virus vectors usable in gene transfer. Recently, adenovirus vectors have attracted the largest attention, since they enable gene transfer into cells which are not under division and can be easily concentrated to a level of about 10¹⁰. Recent studies indicate that genes can be transferred *in vivo* at a high ratio into airway epithelial cells, hepatic cells, muscular cells, etc. by using these adenovirus vectors. On the other hand, such an adenovirus vector essentially has a characteristic that a foreign gene is not integrated into the genomic DNA of the target cells. After treating the target cells with the vector, therefore, the effects of the gene transfer can be sustained only for several weeks or several months at the longest. Accordingly, it is necessary to repeat the gene transfer, which brings about some problems such as increased physical and mental loads on the patient, a decrease in the gene transfer efficiency due to the appearance of anti-adenovirus antibody, etc.

In contrast, adeno-associated virus (AAV) vectors are characterized in that a foreign gene is integrated into the genomic DNA of the target cells and the vectors have neither pathogenicity nor cytotoxicity [Kotin R.M., Hum. Gene Ther., 5, 793 - 901 (1994); Nienhuis A.W. et al., Viruses and Bone Marrow, Dekker Inc., 353 - 414 (1993)]. Moreover, the ITR (inverted terminal repeat) thereof, which is needed in packaging viral particles and gene integration into genomic DNA, has no promotion activity for gene expression. Thus, the gene expression can be arbitrarily switched on/off by setting an appropriate inner promoter, or a tissue-specific promoter can be employed. In the case of the AAV vectors, use can be made of hosts over a wide range, which makes this vector applicable to various target cells/diseases. Owing to these characteristics, the AAV vectors are expected to be novel virus vectors, i.e., a substitute for the MoMLV vectors. It is also found that AAV of wild type is integrated into a definite site in the 19th chromosome [Samulski R.J., et al., EMBO J., 10, 3941-3980 (1991)]. Thus AAV vectors attract public attention as vectors capable of targeting the gene integration site.

The MoMLV vectors, adenovirus vectors and AAV vectors described above hold common characteristic, i.e., having no host specificity. This characteristic means that a number of cells *in vivo* can be treated with these virus vectors. That is to say, use of these virus vectors would contribute to the cure of various diseases.

As described above, these virus vectors are characterized by being free from host specificity, but on the other hand, this characteristic makes it difficult to administer these vectors to living organisms. When these virus vectors are employed in therapy in practice, various methods are necessary in the administration thereof. A known process for treating hemocytes comprises taking out the cells to be treated from the body and, after the completion of the gene transfer, putting the cells back into the body again (i.e., *ex vivo* gene transfer). In the case of cells lodged in an organ or a tissue, a virus vector is topically administered into the organ or tissue to be treated. In the former case, specific equipment is required, which restricts the facilities where the treatment can be carried out. On the other hand, the latter case suffers from a problem that a surgical operation should be performed in some cases for the topical administration. When a disease is to be treated by inducing cellular death, in particular, it is essentially required from the viewpoint of safety to transfer a gene exclusively into the target cells. The above-mentioned virus vectors fail to satisfy this requirement.

In gene therapy for AIDS, CD4-positive T lymphocytes are the target cells. In recent years, there have been developed a number of drug genes aiming at the gene therapy for AIDS. Thus it is urgently required to develop a system by which such a gene can be efficiently and specifically delivered to CD4-positive T lymphocytes. In an attempt to treat AIDS through the introduction of a gene capable of inducing cellular death, in particular, the gene should be transferred exclusively into CD4-positive T lymphocytes from the viewpoint of safety. However, virus vectors which have been clinically employed hitherto (i.e., MoMLV vectors, adenovirus vectors, AAV vectors, ec.) have no tissue-specificity as discussed above and thus cannot satisfy this requirement.

### DISCLOSURE OF THE INVENTION

The present invention, which has been completed under the above-mentioned circumstances, aims at providing a system by which a gene capable of directly or indirectly damaging cells is efficiently and specifically transferred into CD4-positive T lymphocytes.

To achieve the above-mentioned object, the present inventors have conducted extensive studies and, as a result, found that a cytotoxic gene can be efficiently and specifically transferred into CD4-positive cells with the use of a human immunodeficiency virus vector and the gene thus transferred is expressed exclusively in HIV-infected cells, thus completing the present invention.

Accordingly, the present invention provides a recombinant human immunodeficiency virus vector which carries a DNA sequence encoding a cytotoxin.

### BRIEF DESCRIPTION OF THE DRAWINGS

Fig. 1 is a model view which shows the structure of a recombinant plasmid HXTKN.

Fig. 2 is a model view which shows the structure of a helper plasmid CGPE(-).

Fig. 3 is a model view which shows the structure of a vector plasmid HXN.

Fig. 4 is a diagram (a photograph showing the morphology of an organism) which shows G418-resistant colonies obtained by gene transfer with the use of a recombinant virus.

Fig. 5 is a diagram (an electrophoretic pattern) which shows the result of Southern blotting performed after digestion of treating genomic DNA with Xho I.

Fig. 6 is a diagram (an electrophoretic pattern) which shows the result of Southern blotting performed after digestion of genomic DNA with Sca I.

Fig. 7 is a model view which shows the structure of a plasmid BTA/LA encoding Tat.

Fig. 8 is a diagram (an electrophoretic pattern) which shows the result of Northern blotting indicating the Tat-dependent RNA expression.

### BEST MODE FOR CARRYING OUT THE INVENTION

The virus vector to be used in the present invention is a human immunodeficiency virus vector. It is known that human immunodeficiency virus (HIV) infects specifically human CD4-positive T lympocytes. This is because the envelope protein gp120 of HIV specifically binds to CD4 protein located on the surface of CD4-positive T lympocytes at the infection. This specific infection system of HIV is utilized in the virus vector according to the present invention [Shimada T., et al., J. Clin. Invest., 88, 1043 (1991)].

The recombinant human immunodeficiency virus vector of the present invention is prepared by integrating a DNA sequence encoding a cytotoxin into an HIV vector. The term "cytotoxin" means a substance which acts as a cytotoxic gene in cells. Examples thereof include enzyme genes capable of converting a nontoxic prodrug into a substance with cytotoxicity (for example, thymidine kinase catalyzing monophosphorylation of GCV so as to induce cellular death) and genes of various toxins directly damaging cells (for example, diphtheria toxin, tetanus toxin, cholera toxin). It is preferable to use thymidine kinase, still preferably thymidine kinase originating in human herpes simplex virus, as an enzyme gene to be employed as a cytotoxin. As a toxin, it is preferable to use diphtheria toxin.

The cytotoxin of the present invention can be integrated into the HIV vector by using gene recombination techniques publicly known in the field of molecular biology.

In a preferable embodiment, the recombinant human immunodeficiency virus vector of the present invention can be constructed so that no inner promoter exists in the upstream of the DNA sequence encoding a cytotoxin and thus this gene is driven exclusively by HIV LTR. Thus, the gene undergoes expression exclusively in the presence of the Tat protein originating in HIV. Even though the gene is transferred into CD4-positive T lympocytes not infected with HIV, therefore, the gene is not expressed and thus no cellular death is induced thereby. In this embodiment, HIV-infected cells can be selectively damaged and, therefore, it can be expected that a highly specific treatment can be established thereby.

In the present invention, a preferable virus vector for specific gene transfer may be prepared in, for example, the following manner.

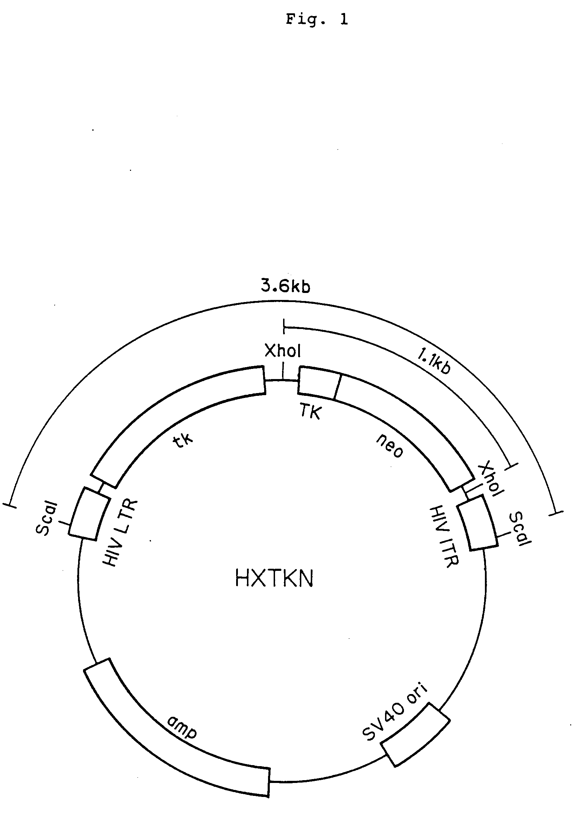

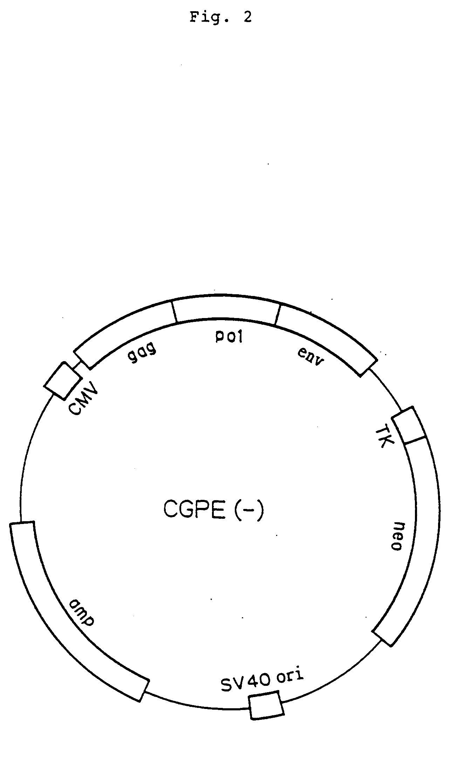

The virus vector of the present invention can be obtained by the cotransfection of COS cells with a helper plasmid shown in Fig. 2 (i.e., a plasmid successively containing, starting from the 5'-end toward the 3'-end, a cytomegalovirus promoter, genes encoding gag, pol and env of HIV and lacking any packaging signal, a promoter of thymidine kinase originating in herpes virus, and a neomycin resistance gene and having been inserted into a plasmid vector containing an ampicillin resistance gene and the replication origin of SV40) and a vector plasmid shown in Fig. 1 (i.e., a plasmid having the long terminal repeats (LTRs), between which a gene for damaging cells, a thymidine kinase promoter originating in herpes virus and a neomycin resistance gene have been successively inserted starting from the 5'-end toward the 3'-end, and having inserted into a plasmid vector containing an ampicillin resistance gene and the replication origin of SV40).

The HIV vector of the present invention thus obtained not only enables gene transfer specifically into human CD4-positive T lymphocytes but also can selectively damage HIV-infected cells alone. Because of having been driven by HIV LTR, the cytotoxic gene in the present invention is expressed exclusively in the presence of the Tat protein originating in HIV. Even though the gene is transferred into CD4-positive T lymphocytes not infected with HIV, therefore, no cellular death is induced thereby. By using the gene transfer method with the use of this HIV vector, a foreign gene is integrated into the genomic DNA in a stable state. After cell division, the daughter cells succeed to the gene thus integrated. Namely, this method is highly useful in the prevention of the infection. That is to say, the HIV vector of the present invention is a vector for gene therapy seemingly having an extremely wide application range by which the various inconveniences of the conventional MoMLV, adenovirus and AAV vectors can be solved.

### EXAMPLES

To further illustrate the present invention in greater detail, and not by way of limitation, the following Examples will be given.

### Example 1: Construction of plasmid

All plasmids were constructed in accordance with genetic engineering procedures commonly employed in the art. The recombinant plasmid HXTKN was constructed by inserting a plasmid, which had successively from the 5'-end toward the 3'-end, HIV LTR, a thymidine kinase gene originating in human herpes simplex virus (tk), a promoter of the thymidine kinase gene originating in human herpes simplex virus (TK), a neomycin resistance gene (neo) and HIV LTR, into a plasmid vector having the replication origin of SV40, as shown in Fig. 1 [Shimada T., et al., J. Clin. Invest., 88, 1043 (1991)].

### Example 2: Preparation of recombinant virus

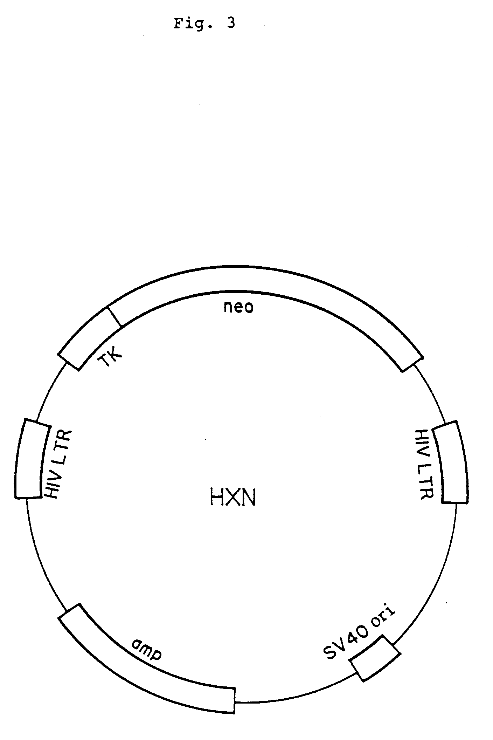

10 µg of HXTKN obtained in the above Example 1 was mixed with 10 µg of a helper plasmid CGPE (-) wherein a gene construction consisting of a cytomegalovirus promoter in the upstream of the group of genes containing the gag, pol and env genes in the HIV genomic sequence and lacking any packaging signal had been inserted into an expression vector containing an ampicillin resistance gene and the replication origin of SV40, as shown in Fig. 2. Next, sterile purified water and an aqueous solution of calcium chloride were added to the mixture so as to adjust the total volume to 0.5 ml. The resulting mixture was dropped into 0.5 ml of an HBSP buffer under shaking and allowed to stand at room temperature for 30 minutes to thereby give a plasmid/calcium phosphate coprecipitate. This coprecipitate was added to the culture medium of COS cells which had been incubated in a culture dish (9 cm) up to about 70 % confluence. After incubation in a CO₂ incubator for 4 hours, the culture medium was replaced by a fresh one and the incubation was continued for additional 2 days. The culture supernatant thus obtained was referred to as a virus solution. As negative controls, virus solutions were prepared with the use of a group transfected with 10 µg of CGPE (-) alone. As positive controls, on the other hand, virus solutions were prepared by using a group cotransfected with 10 µg of a vector plasmid HXN (Fig. 3) [Shimada T., et al., J. Clon. Invest., 88, 1043 (1991)] and 10 µg of CGPE (-).

### Example 3: Transduction of cells with recombinant virus and cloning of drug-resistant strain

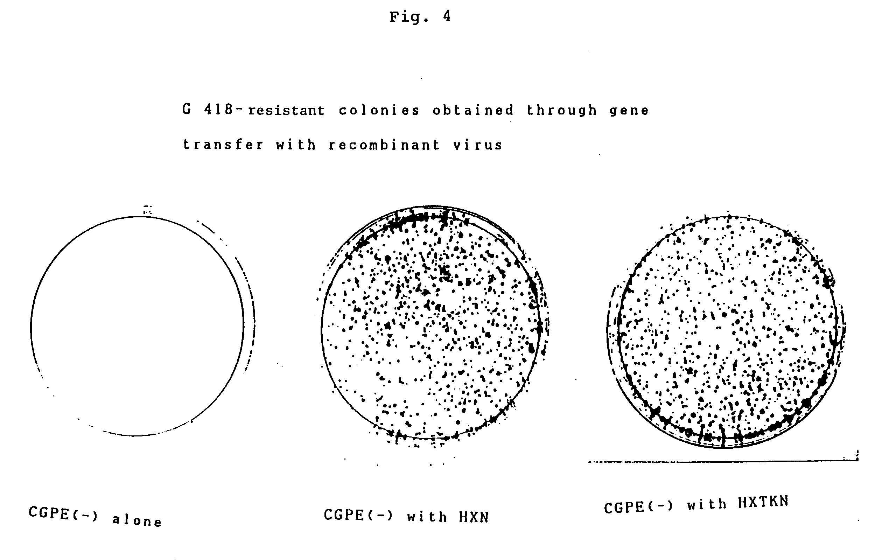

CD4-positive HeLa cells were inoculated at a density of 2 x 10⁵ per culture dish (60 mm) and incubated overnight. 3 ml of each virus solution obtained in Example 2 was added thereto together with 30 µg of polybrene (transduction). After standing in a CO₂ incubator for 2 days, the cells were peeled off from the dish with the use of a mixture of trypsin/EDTA and inoculated again into another culture dish (90 mm). After incubation in the CO₂ incubator for 6 hours, G-418 was added so as to give a concentration of 750 µg/ml of the culture medium. After incubation for an additional 10 days, G-418, i.e., an analog of neomycin was added to the culture medium so as to give a final concentration of 1,000 µg/ml. After incubation for an additional 10 days, colonies acquiring the resistant to G-418 were obtained. These colonies were stained with Crystal Violet. Fig. 4 shows the results. In the negative control group, no recombinant virus vector was formed and thus the cells were all exterminated. In the group cotransfected with CGPE (-) and HXN and the group cotransfected with CGPE (-) and HXTKN, in contrast thereto, a number of drug-resistant colonies were observed. These two groups were almost comparable to each other in terms of the number of colonies. These results indicate that the insertion of the tk gene into the genomic DNA of the recombinant HIV vector causes no decrease in the titer of the recombinant HIV vector and the tk gene transferred into the cells shows no cytotoxicity. Some of the colonies were peeled off from the culture dish with the use of a mixture of trypsin-EDTA and each inoculated into another culture dish for the subsequent analysis.

### Example 4: Extraction of genomic DNA from transduced CD4-positive Hela cells and confirmation of gene transfer by Southern blotting

Cells reaching confluence in a culture dish (90 mm) were peeled off from the dish by treating with a mixture of trypsin-EDTA and lysed by adding a surfactant thereto. After adding proteinase K to give a final concentration of 200 µg/ml, incubation was carried out at 37 °C for 3 hours. After deproteinization by extracting with phenol/chloroform, the residue was dialyzed against Tris/EDTA buffer overnight. Next, sodium chloride (final concentration: 0.2 M), RNase A (final concentration: 100 µg/ml) and proteinase K (final concentration: 100 µg/ml) were added and incubation was carried out at 37 °C for 3 hours. After extracting with phenol/chloroform, the culture was dialyzed against Tris/EDTA buffer again. After 2 days, the dialysis was completed and the genomic DNA was obtained.

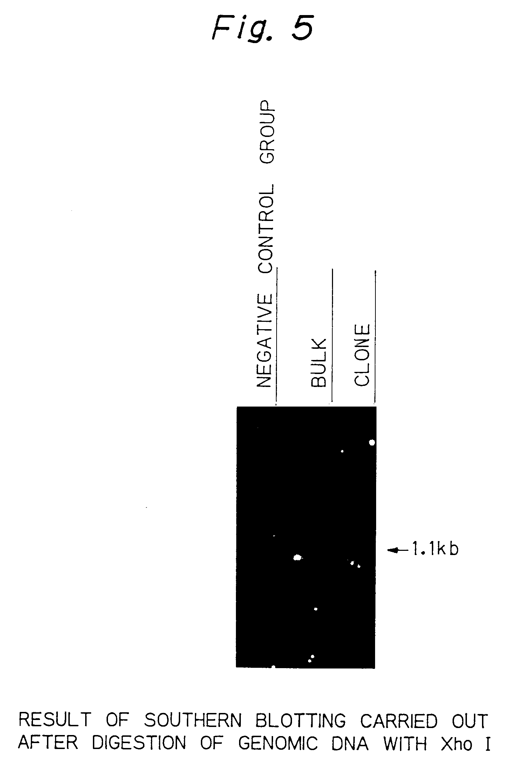

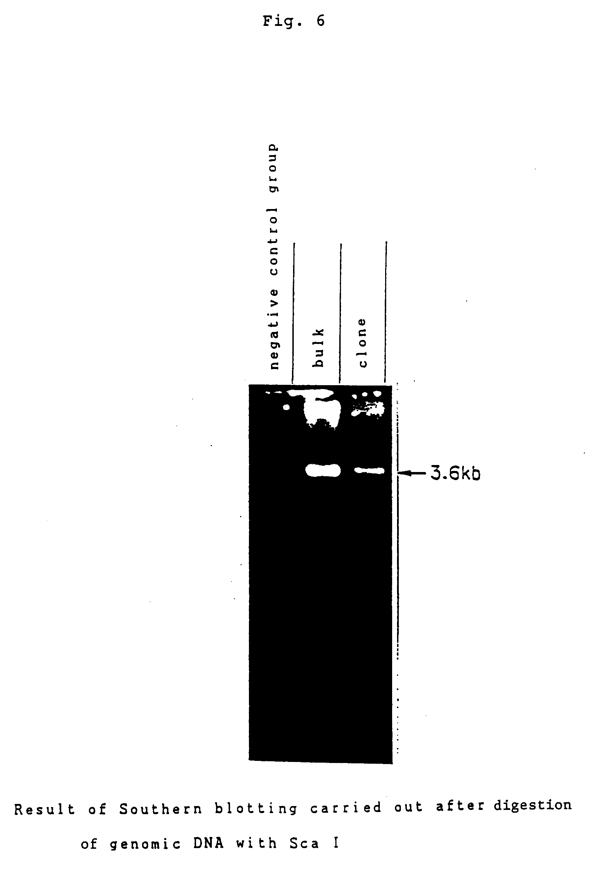

Southern blotting was performed in a conventional manner. 10 µg of the genomic DNA was digested with a restriction enzyme Xho I or ScaI. As a negative control group, CD4-positive HeLa cells not treated with the recombinant virus were subjected to the same procedure. As a probe, use was made of a neo gene (600 bp) labeled with ³²P. Figs. 5 and 6 show the results. The clone means a single strain acquiring the resistant to G-418, while the bulk means a mixture of 50 clones. In the group digested with Xho I, a radioactivity was observed at 3.6 kb, which proves that the recombinant gene had been inserted into the genomic DNA by the recombinant virus vector of the present invention without causing any rearrangement.

### Example 5: Confirmation of Tat-dependent RNA expression

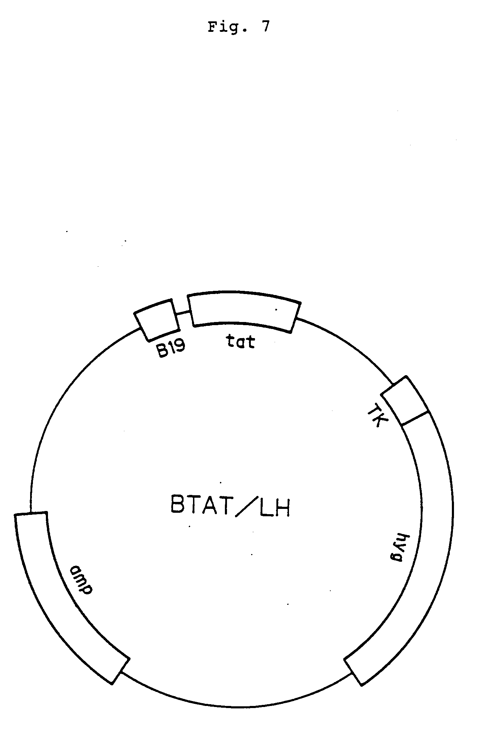

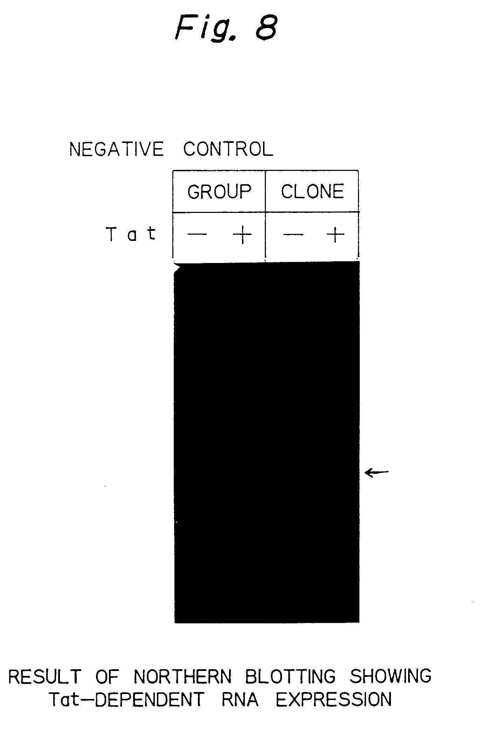

The cell strain (clone) obtained in Example 3 was transfected with 10 µg of a plasmid BTAT/LH encoding Tat (shown in Fig. 7) by the calcium phosphate method. As a negative control group, CD4H cells not treated with the recombinant virus were also transfected in the same manner. After being allowed to stand in a CO₂ incubator for 2 days, the cells were peeled off from the culture dish and treated with a cell lytic solution containing guanidine isothiocyanate. Then proteins and DNA were eliminated therefrom by the cesium chloride gradient centrifugation so that RNA alone was separated. After electrophoresis, Northern blotting was carried out in a conventional manner. As a probe, use was made of a tk gene (600 bp) labeled with ³²P. As Fig. 8 shows, no expression of the tk gene was observed in the negative control group and the clone not transfected with BTAT/LH. In contrast thereto, it was confirmed that the RNA was expressed depending on Tat in the clone transfected with BTAT/LH.

## Claims

1. A recombinant human immunodeficiency virus vector which carries a DNA sequence encoding a cytotoxin.

2. A recombinant human immunodeficiency virus vector as claimed in Claim 1, wherein said cytotoxin is thymidine kinase.

3. A recombinant human immunodeficiency virus vector as claimed in Claim 1, wherein no internal promoter exists in the upstream of the DNA sequence encoding a cytotoxin and said gene is driven exclusively by HIV LTR.

4. A recombinant human immunodeficiency virus vector as claimed in Claim 1, wherein an internal promoter for driving the DNA sequence encoding a cytotoxin is located.
